Europäisches Patentamt

European Patent Office

Office européen des brevets

(19)

(11) Veröffentlichungsnummer : **0 160 115
B1**

(12) **EUROPÄISCHE PATENTSCHRIFT**

(45) Veröffentlichungstag der Patentschrift :
19.08.87

(21) Anmeldenummer : 84104889.5

(22) Anmeldetag : 02.05.84

(51) Int. Cl.⁴ : **A 23 K   1/16**, A 23 K   1/17,
C 07 D241/42, C 07 D241/52

(54) **Wachstumsstimulierender Futterzusatz für Nutztiere.**

(43) Veröffentlichungstag der Anmeldung :
**06.11.85 Patentblatt 85/45**

(45) Bekanntmachung des Hinweises auf die Patenterteilung : **19.08.87 Patentblatt 87/34**

(84) Benannte Vertragsstaaten :
**AT BE CH DE FR GB IT LI NL SE**

(56) Entgegenhaltungen :
EP-A- 0 019 809
DE-A- 2 541 459
US-A- 4 225 604
**JOURNAL OF MEDICINAL CHEMISTRY Band 19, Nr. 2, 1976; M.L. EDWARDS et al. "Beta-Lactam antibiotics with N-oxide side chains. 1. Quinoxaline N-oxides", Seiten 330-333**

(73) Patentinhaber : **SPOFA Spojené Podniky Pro Zdravotnickou Vyrobu
Husinecká 11 a
Prag 3 (CS)**

(72) Erfinder : **Sevcik, Bohumil, Prof. MVDr DrSc
Chotoun 79
Pohori (CS)**
Erfinder : **Strakova, Jana, MVDr
Nad lesnim divadlem 1116
Praha 4 (CS)**
Erfinder : **Chromik, Jindrich, Dipl.-Ing.
Na poustkách 2131
Praha 4 (CS)**
Erfinder : **Hebky, Jaromir, Dipl.-Ing. Dr.
Zdanova 24
Praha 6 (CS)**
Erfinder : **Dvorák, Miroslav, RNDr
Ustrasická 15
Planá nad Luznici (CS)**

(74) Vertreter : **Patentanwälte Beetz sen. - Beetz jun.
Timpe - Siegfried - Schmitt-Fumian
Steinsdorfstrasse 10
D-8000 München 22 (DE)**

**Beschreibung**

Die Erfindung betrifft einen synergistisch wirksamen wachstumsstimulierenden Futterzusatz mit einem Gehalt an 2-Formylchinoxalin-1,4-dioxid-cyanoacetyl-hydrazon (generische Bezeichnung Cyadox), in Kombination mit dem Antibioticum Tylosin und/oder dem Sulfonamid Phthalazol sowie die Verwendung dieses Futterzusatzes zur Anreicherung des Futters bei der Zucht von Nutztieren, wie z. B. Schweinen, Kälbern und Geflügel. Der erfindungsgemäße Futterzusatz kann zur Steigerung der Gewichtszunahme, und zur Verbesserung der Futtermittelausnutzung eingesetzt werden.

Wie bekannt (CS-A 195 508, DE-B 2 829 580, US-A 4 225 604), wirkt Cyadox als starker Stimulator des Wachstums von Nutztieren und hemmt Gram-positive und Gram-negative Mikroorganismen sowie die Kokzidioseerreger. Ein besonderer Vorteil ist, daß diese Verbindung nahezu nicht toxisch ist, da ihre 50 %-Letaldosis $LD_{50}$ bei Ratten 15 g/kg p. o. beträgt.

Es erwies sich nun überraschenderweise, dass die erwähnten Wirkungen von Cyadox auf das Wachstum und den Allgemeinzustand der Tiere durch Zusatz des Antibioticums Tylosin und/oder des Sulfonamids Phthalazol (N-(4-(Thiazol-2-yl-sulfamoyl)-phenyl)-phthalamsäure), die an sich keine Wachstumsförderung bewirken, in ausgeprägter Weise überproportional gesteigert werden, da die beobachtete Gesamtwirkung wesentlich höher ist, als aufgrund der bekannten Wirksamkeit der Einzelstoffe zu erwarten war. In der erwähnten Kombination wird die günstige Wirkung von Cyadox in unerwarteter Weise gesteigert, indem die genannten Zusatzkomponenten sowohl zur Wachstumsförderung als auch zur Unterdrückung pathogener Mikroorganismen durch Cyadox synergistisch beitragen. Dieser Synergismus ist, wie auch z. B. die äußerst geringe Toxizität und erhöhte Beständigkeit, auf die außergewöhnlichen Eigenschaften von Cyadox zurückzuführen.

Der erfindungsgemässe wachstumsstimulierende Futterzusatz für Nutztiere ist entsprechend gekennzeichnet durch ein Gemisch von 2-Formylchinoxalin-1,4-dioxid-cyanoacetyl-hydrazon mit dem Antibioticum Tylosin und/oder dem Sulfonamid Phthalazol.

Wenn man beispielsweise bei frühabgesetzten Ferkeln einzelne Tiergruppen jeweils entweder mit Cyadox allein, mit Tylosin allein oder mit einer Kombination beider Wirkstoffe in der gleichen Dosis von z. B. 100 mg/kg Futtermittel behandelt, sind bei der Kombination sowohl die Gewichtszunahme als auch die Futtermittelausnutzung signifikant höher als die Summe dieser Wirkungen der einzeln verabfolgten Komponenten. Ähnliche Resultate ergeben sich u. a. bei der Futtermittelanreicherung mit Cyadox-Phthalazol-Kombinationen bei Vormastschweinen von etwa 50 kg Gewicht, z. B. unter Zusatz von je 100 mg/kg Cyadox und Phthalazol, bei unabhängiger bzw. kombinierter Verabreichung. Auch hier ist die Wirkung der Kombination im Vergleich mit der unbehandelten Kontrollgruppe wesentlich stärker als die Summe der Wirkung der Einzelkomponenten. Es handelt sich hier also um ein echtes synergistisches Zusammenwirken, das aufgrund der bisher vorliegenden Kenntnisse nicht vorauszusehen war.

Im erfindungsgemäßen Futterzusatz liegt das Tylosin in einer geeigneten Darreichungsform vor, z. B. als freie Base oder als Additionssalz mit organischen oder anorganischen Säuren, als Komplexsalz oder Metallchelat oder auch in Form eines getrockneten Fermentationsmediums bzw. eines davon abgetrennten getrockneten, tylosinhaltigen Mycels des Produktionsstamms.

Entsprechende Säureadditionssalze sind z. B. das Hydrochlorid, Sulfat, Phosphat oder Tartrat. Die Sulfonamidkomponente Phthalazol kann ferner in analoger Weise als entsprechende freie Verbindung sowie als Additionssalz mit organischen oder anorganischen Säuren vorliegen.

Infolge der sehr geringen Toxizität von Cyadox sowie Tylosin und Phthalazol besteht bei der Anwendung des erfindungsgemäßen Futterzusatzes keinerlei Gefahr einer Schädigung bei eventueller unbeabsichtigter Überdosierung im damit angereicherten Mischfutter. Auch bei Anwendung als Arzneimittel in der Veterinärmedizin lassen sich die erforderlichen Dosen zuverlässig und ohne Schädigungsrisiko verabreichen.

Der synergistisch wirksame wachstumsstimulierende Futterzusatz nach der Erfindung kann Cyadox einerseits und Tylosin und/oder Phthalazol andererseits praktisch in jedem beliebigen Mengenverhältnis, zweckmäßig z. B. in einem Mengenverhältnis im Bereich von 1 : 10 bis 10 : 1, enthalten. Zur Erzielung optimal ausgewogener Wirkungen und des stärksten Synergismus ist jedoch in den meisten Fällen ein ausgeglichenes Verhältnis von etwa 1 : 1 besonders empfehlenswert. Der erfindungsgemäße Futterzusatz kann neben Cyadox gleichzeitig sowohl Tylosin als auch Phthalazol enthalten. Bei der Zubereitung des damit angereicherten Fertigfutters wird der Futterzusatz am besten als Vormischung (Prämix) zu nutritiv ausgewogenen Futtergemischen mit dem erforderlichen Gehalt an Biofaktoren in einer Menge von etwa 20 bis 500 g jeder Komponente pro Tonne Fertigfutter zugegeben. Die Komponenten können ferner auch in einer geeigneten veterinärmedizinischen Arzneimittelform in Tagesdosen von je etwa 1 bis 100 mg pro Kilogramm Lenbendgewicht verabfolgt werden.

Die zur Verabreichung des erfindungsgemäßen Futterzusatzes geeigneten Arzneimittelformen können fest oder flüssig sein und z. B. als Granulate, Kapseln, Pillen, perorale Pasten, Suspensionen udgl. vorliegen. Sie können ferner verträgliche Lösungs- und Verdünnungsmittel sowie Träger- und Hilfsstoffe organischen oder anorganischen Ursprungs enthalten, z. B. gemahlenen Kalkstein, Kaolin, Talk, Stärke, Zucker, Cellulose, ggfs. zerriebene Pflanzenreste und ähnliche für die Zubereitung von veterinärmedizinischen Arzneimitteln verwendete Zusatzstoffe, ferner übliche Excipientien und er-

2

forderlichenfalls weitere Wirkstoffe. Zu den Excipientien gehören insbesondere pharmazeutisch geeignete grenzflächenaktive Stoffe, wie z. B. übliche kationaktive, anionaktive oder nichtionische Tenside und deren Gemische mit guten Netzmittel-, Dispergiermittel- bzw. Emulgiermitteleigenschaften. Diese grenzflächenaktiven Stoffe, die in zweckmäßiger Weise unter der Vielzahl der bekannten Verbindungen ausgewählt werden, können mit Vorteil den Prämixen und Futtergemischen mit beigemengt werden. Diese Tenside werden vorzugsweise in einer Menge von 0,5 bis 10,0 kg pro Tonne Fertigfutter zugesetzt.

Die Darreichungsformen des erfindungsgemäßen Futterzusatzes werden wie übliche veterinärmedizinische Arzneimittelformen, Prämixe bzw. Wirkstoffkonzentrate und mit Biofaktoren angereicherte Futtergemische auf bekannte Weise hergestellt, insbesondere durch inniges Vermischen oder Zermahlen der Wirkstoffe mit den Zusatzkomponenten, wie z. B. Lösungsmitteln, Trägern und grenzflächenaktiven Stoffen.

Der erfindungsgemäße Futterzusatz stimuliert das Wachstum von Nutztieren in wesentlich höherem Ausmaß als seine Einzelkomponenten und verbessert die Futtermittelausnutzung. Er kann ferner mit Vorteil zur Vorbeugung bzw. Therapie von systemischen Tiererkrankungen, insbesondere von Durchfallerkrankungen, wie Enteritiden und Salmonellosen und von Kokzidiose, eingesetzt werden. Dieser Futterzusatz verbessert den Allgemeinzustand der Tiere, verringert die Verluste durch Sterben in frühen Lebensstadien und erhöht so den wirtschaftlichen Nutzen in der Nutztierzucht, insbesondere bei der Großzucht von Schweinen, Rindern und Geflügel.

Die nachfolgenden Beispiele erläutern Anwendungsweise und Wirkungen des erfindungsgemäßen Futterzusatzes. Die Prozentangaben sind massen bezogen.

Beispiel 1

Frühabgesetzte Ferkel (Mischlinge des weißen edlen Landschweins x Landrasse), die innerhalb einer Zeitspanne von 6 Tagen geboren waren, wurden am 5. Tage nach dem Absetzen aus der Gebärstation in die Versuchsanlage gebracht. Die Ferkel wurden in Gruppen von je 8 bis 12 Tieren in Boxen untergebracht, die mit Rostböden versehen und durch hölzerne Bodenplatten beheizt waren. Nach 7 Tagen Anpassungszeit wurde der eigentliche Mastversuch begonnen. Über die gesamte Versuchsdauer von 4 Wochen wurde in allen Gruppen das Futtergemisch ČOS 2 für früh abgesetzte Ferkel verfüttert, das durch Zusatz der entsprechenden Prämixe jeweils mit folgenden Wirkstoffen angereichert wurde :

Gruppe I (Kontrollgruppe) : Futtergemisch allein ohne Zusatz ;
Gruppe II : Cyadox, 100 ppm (100 mg pro kg Futtergemisch) ;
Gruppe III : Roh-Tylosin, 100 ppm reine Tylosinbase ;
Gruppe IV : Cyadox und Roh-Tylosin, je 100 ppm.

Zu Beginn des Versuchs und am Versuchsende (28. Tag) wurden die Tiere individuell gewogen. Der Futterverbrauch pro Gruppe wurde täglich ermittelt und daraus die Futtermittelausnutzung, d. h. der Futterverbrauch pro Zuwachseinheit, berechnet. Ebenso wurde der Allgemeinzustand der Tiere täglich verfolgt. Die Versuchsergebnisse sind in Tabelle 1 zusammengefaßt.

Tabelle 1

| Gruppe | I | II | III | IV |
|---|---|---|---|---|
| Behandlung (Wirkstoffgehalt, ppm) | Kontrolle 0 | Cyadox 100 | Tylosin 100 | Cyadox (100) + Tylosin (100) |
| Anzahl der Tiere | 12 | 12 | 11 | 5 x 8 |
| Anfangsgewicht, $\bar{x}$ (kg) | 10,30 | 10,28 | 9,13 | 7,10 |
| Zuwachsindex (%) | 100,0 | 109,8 | 125,9 | 150,6 |
| Futtermittel-Ausnutzung, $\bar{x}$ | 2,0 | 1,99 | 1,90 | 1,6¹ |
| Ausnutzungsindex (%) | 100,0 | 99,5 (-0,5) | 92,2 (-7,8) | 84,3 (-15,7) |

Ähnliche Resultate ergaben sich unter Verwendung von Cyadox zusammen mit Tylosin zur Anreicherung von Futtergemischen für Schweine, Kälber und Broilerhühner.

Beispiel 2

Die Versuche wurden in einem Schweinemastbetrieb durchgeführt, bei dem ständig bei 5 bis 15 % der Vormatschweine (Rasse BU × PC + L) von etwa 50 kg Gewicht, die in Koben zu 10 bis 15 Tieren untergebracht waren, blutige Durchfälle auftraten. Histologisch und mikrobiologisch wurde als Erreger der Durchfallerkrankung Vibrio coli festgestellt.

Die Versuchstiere wurden in 4 Gruppen aufgeteilt und während der gesamten Versuchsdauer von 14 Tagen mit dem Vormastschweine-Futtergemisch SOL unter folgender Wirkstoffzugabe behandelt :

Gruppe (Kontrollgruppe) : Futtergemisch allein ;
Gruppe II : Phthalazol, 100 ppm ;
Gruppe III : Cyadox, 100 ppm ;
Gruppe IV : Cyadox und Phthalazol, je 100 ppm.

Bei den Gruppen III und IV traten keine Anzeichen blutiger Durchfälle auf (Heilungsgrad 100 %). Dagegen überdauerte in den Gruppen I (Kontrollgruppe) und II (Phthalazol allein) das Krankheitsbild noch bei 20 bis 30 % der Tiere. Aus beiden letztgenannten Gruppen mußten am Versuchsende insgesamt 20 Schweine, und zwar 15 aus Gruppe I und 5 aus Gruppe II, wegen unbefriedigenden Allgemeinzustands notgeschlachtet werden. Die Versuchsergebnisse sind in Tabelle 2 zusammengefaßt.

Ähnliche Resultate ergaben sich bei Verwendung von Phthalazol zusammen mit Cyadox zur Anreicherung von Futtergemischen für Schweine, Kälber und Hühner.

Tabelle 2

| Gruppe | I | II | III | IV | |
|---|---|---|---|---|---|
| Behandlung (Wirkstoffgehalt im Futtermittel in ppm) | Kontrolle (0) | Phthalazol (100) | Cyadox (100) | Cyadox + Phthalazol (100) | (100) |
| Anzahl der Schweine | 73 | 20 | 12 | 74 | |
| Anfangsgewicht $\bar{x}$ (kg) | 50,48 | 50,62 | 47,80 | 53,13 | |
| Endgewicht $\bar{x}$ (kg) | 59,43 | 59,61 | 57,90 | 64,93 | |
| Gesamtzuwachs $\bar{x}$ (kg) | 8,95 | 8,99 | 10,10 | 11,80 | |
| Tageszuwachs (g) | 639 | 642 | 721 | 843 | |
| Zuwachsindex (%) | 100 | 100,5 | 112,8 | 131,8 | |

**Patentansprüche**

1. Wachstumsstimulierender Futterzusatz für Nutztiere auf der Basis von 2-Formylchinoxalin-1,4-dioxid-cyanoacetylhydrazon (Cyadox), gekennzeichnet durch ein Gemisch von 2-Formylchinoxalin-1,4-dioxid-cyanoacetylhydrazon mit Phthalazol, ggfs. in Form eines Additionssalzes, und/oder Tylosin, ggfs. in Form eines Additionssalzes, Komplexsalzes oder Metallchelats oder in Form eines tylosinhaltigen getrockneten Fermentationsmediums oder eines davon abgetrennten tylosinhaltigen Mycels eines entsprechenden Produktionsstamms.

2. Futterzusatz nach Anspruch 1, gekennzeichnet durch ein Mengenverhältnis von Cyadox zu Tylosin und/oder Phthalazol von 0,1 bis 10.

3. Wachstumsstimulierende Futterzubereitung, gekennzeichnet durch einen Futterzusatz nach Anspruch 1 oder 2 mit einem Wirkstoffgehalt von je 20 bis 500 g/t neben üblichen Futter-, Hilfs- und/oder Trägerstoffen.

4. Futterzubereitung nach Anspruch 3, gekennzeichnet durch den Gehalt eines physiologisch verträglichen Tensids in einer Menge von 0,5 bis 10,0 kg/t.

5. Verwendung des Futterzusatzes nach Anspruch 1 oder 2 bzw. der Futterzubereitungen nach Anspruch 3 oder 4 zur Steigerung der Gewichtszunahme und zur Förderung der Futtermittelausnutzung von Nutztieren, insbesondere in der Großzucht.

**Claims**

1. A growth-stimulating feed additive for economic animals on the basis of 2-formyl-quinoxaline-1,4-dioxide-cyanoacetyl hydrazone (cyadox), characterized by a mixture of 2-formyl-quinoxaline-1,4-dioxide-cyanoacetyl hydrazone and phthalazole, optionally in the form of an addition salt, and/or tylosine, optionally in the form of an addition salt, complex salt, or metal chelate, or in the form of a tylosine-containing dried fermentation medium, or of a tylosine-containing mycellium of a corresponding production strain separated therefrom.

2. Feed additive according to claim 1, characterized by a quantitative ratio of cyadox to tylosine and/or phthalazole of 0.1 to 10.

3. Growth stimulating feed preparation characterized by a feed additive according to claim 1 or 2 with a content of active ingredient of 20 to 500 g/t each besides conventional feed, adjuvant, and/or substrate materials.

4. Feed preparation according to claim 3, characterized by the content of a physiologically tolerable tenside in an amount of 0.5 to 10.0 kg/t.

5. The use of the feed additive according to claim 1 or 2, or of the feed preparations according to claim 3 or 4, for enhancing weight gain and for promoting feed utilization of economic animals, especially during the rearing period.

**Revendications**

1. Additif alimentaire, stimulant la croissance, pour animaux de rapport, à base de 2-formylquinoxaline-1,4-dioxyde-cyanoacétylhydrazone (Cyadox) caractérisé par un mélange de 2-formylquinoxaline-1,4-dioxyde-cyanoacétylhydrazone et de phtalazole, éventuellement sous la forme d'un sel d'addition, et/ou de tylosine, éventuellemnt sous la forme d'un sel d'addition, d'un sel complexe ou d'un chélate métallique, ou sous la forme d'un milieu de fermentation séché contenant de la tylosine, ou d'un mycélium, séparé de ce milieu et contenant de la tylosine, d'une souche de production correspondante.

2. Additif alimentaire selon la revendication, caractérisé par un rapport quantitatif de 0,1 à 10 entre le Cyadox et la tylosine et/ou le phtalazole.

3. Préparation alimentaire stimulant la croissance, caractérisée par un additif alimentaire selon la revendication 1 ou 2, contenant 20 à 500 g/t de chaque principe actif, en plus des aliments, adjuvants et/ou excipients habituels.

4. Préparation alimentaire selon la revendication 3, caractérisée en ce qu'elle contient en une quantité de 0,5 à 10,0 kg/t un tensioactif physiologiquement compatible.

5. Utilisation de l'additif alimentaire selon la revendication 1 ou 2, ou des préparations alimentaires selon la revendication 3 ou 4, pour augmenter le gain de poids et pour améliorer le taux d'utilisation des aliments, en particulier dans les grands élevages.